(19) Europäisches Patentamt / European Patent Office / Office européen des brevets

(11) **EP 3 344 130 B1**

(12) **EUROPEAN PATENT SPECIFICATION**

(45) Date of publication and mention
of the grant of the patent:
**18.11.2020  Bulletin 2020/47**

(21) Application number: **16760087.3**

(22) Date of filing: **03.09.2016**

(51) Int Cl.:
**A61B 5/0225** *(2006.01)*     **A61B 5/022** *(2006.01)*

(86) International application number:
**PCT/EP2016/070787**

(87) International publication number:
**WO 2017/037272 (09.03.2017 Gazette 2017/10)**

(54) **NON-INVASIVE BLOOD PRESSURE MONITORING DEVICE AND METHOD**

VORRICHTUNG ZUR NICHTINVASIVEN BLUTDRUCKÜBERWACHUNG UND VERFAHREN

DISPOSITIF ET PROCÉDÉ DE SURVEILLANCE NON INVASIVE DE LA PRESSION SANGUINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2015  EP 15183620**

(43) Date of publication of application:
**11.07.2018  Bulletin 2018/28**

(73) Proprietor: **Koninklijke Philips N.V.**
**5656 AG Eindhoven (NL)**

(72) Inventor: **AELEN, Paul**
**5656 AE Eindhoven (NL)**

(74) Representative: **Philips Intellectual Property & Standards**
**High Tech Campus 5**
**5656 AE Eindhoven (NL)**

(56) References cited:
JP-A- 2006 129 920     US-A1- 2012 323 128
US-A1- 2014 257 116     US-A1- 2014 316 290
US-A1- 2015 230 718

**Description**

FIELD OF THE INVENTION

The present disclosure relates to the field of vital signs detection, particularly to the field of blood pressure detection. More particularly, the present disclosure, at least in some aspects, relates to refinements of non-invasive blood pressure measurement methods, also referred to as NIBP. More particularly, the present disclosure relates to blood pressure measurement devices that are suitable for measuring a blood pressure during the inflation of a cuff, and to control methods for such blood pressure measurement devices. Generally, non-invasive blood pressure detection may be referred to as methods of and approaches to detect arterial blood pressure in a mediate fashion without the need of obtrusive measures applied to the body of a subject of interest.

BACKGROUND OF THE INVENTION

As opposed to invasive blood pressure measurement, non-invasive blood pressure measurement is a method of measuring the blood pressure of a human body indirectly. The most established non-invasive blood pressure measurement methods require an inflatable cuff to be place around a limb, where the pressure in this cuff is changed to infer blood pressure. Generally, respective devices may be referred to as sphygmomanometers. Within the sphygmomanometers, there are two categories of determining blood pressure.

A first method for non-invasive blood pressure detection is the so-called auscultatory-based method. Auscultation measurements are based on listening to the sounds of the artery during the period that the cuff pressure is changed. These sounds are referred to as so-called Korotkoff sounds, as Korotkoff was the first person that linked these sounds to blood pressure. Sounds are typically measured with a stethoscope that is placed at the brachial artery. First, the cuff is inflated until the flow of the blood in the artery is blocked, which can be observed by the absence of Korotkoff sounds. Then the cuff is deflated. Users (usually medical staff) may determine the blood pressure by listening to the change of the Korotkoff sounds. The pressure at the occurrence of the first sound is usually referred to as the systolic blood pressure. During further deflation, sounds first become louder and decrease in amplitude later on. The pressure at which the last sound is present is usually referred to as the diastolic blood pressure.

A second method for non-invasive blood pressure detection is the so-called oscillometric method. In this method, the systolic and diastolic blood pressure values are dependent on the amplitudes of the pressure oscillations in the blood pressure cuff. The amplitudes of these pressure oscillations are measured at different cuff pressures, where the systolic and diastolic blood pressure values are usually calculated as the cuff pressure where the pressure oscillations are a certain fraction of the maximum of the pressure oscillations. Conventionally, the cuff pressure is changed by first blocking the flow of blood by inflating the cuff. Then, the cuff is deflated slowly during which the oscillation amplitudes are measured. Currently, most of the electronic automatic blood pressure measuring devices measure the blood pressure based on the oscillometric method which has been widely used in clinical practice and in the home situation.

US 2014/0309541 A1 discloses an oscillometric blood pressure measurement device comprising a cuff that, when worn on a blood pressure measurement area, pressurizes an artery in the measurement area at a pressure of a fluid in the cuff; a piezoelectric pump that increases the pressure within the cuff; a deflating unit that reduces the pressure within the cuff; a pressure detection unit that detects a cuff pressure that is the pressure within the cuff; and a control unit, wherein the control unit is arranged to determine an amplitude and a frequency of a voltage applied to the piezoelectric pump; carry out control so that a voltage at the amplitude and frequency determined by the determination unit is applied to the piezoelectric pump; and calculate a blood pressure value based on the cuff pressure detected during inflation when the cuff pressure is increased by the piezoelectric pump. Consequently, US 2014/0309541 A1 describes a blood pressure measurement device that is arranged to analyse the inflation stage so as to derive the blood pressure.

Conventionally, oscillometric blood pressure measurement methods utilize the deflation stage of a measurement wherein the pressure is gradually reduced so as to determine blood pressure indicative signals. Oscillometric blood pressure measurement are basically arranged to derive characteristic blood pressure values such as systolic blood pressure and diastolic blood pressure, in a mediate fashion from measured values and empirical parameters which are typically device-dependent.

Deflation stage based measurement is well established but is at the same time to some extent uncomfortable since the subject (or patient) is exposed to a relatively high cuff pressure for a certain amount of time. Further, since the deflation stage is used, first a defined maximum pressure level needs to be achieved starting from which the deflation procedure may be initiated. Therefore, the patient is exposed to a higher maximum cuff pressure than basically required for the blood pressure measurement as such. Further, the inflation of the cuff takes some time and also the deflation regime takes a considerable amount of time which further increases the discomfort for the patient (or subject).

Inflation stage based blood pressure measurements may reduce the discomfort as the blood pressure measurement may be accomplished in less time as the inflating part of cuff pressure is used rather than the deflating part.

Once the pressure range of interest is passed at the inflation stage, pressure relief may be initiated so as to reduce the overall measurement time and the pressure-based discomfort.

However, also in the domain of inflation-based non-invasive blood pressure measurement methods and devices, there is room for improvement. Generally, an appropriate inflation rate (the inflation rate may be also referred to as cuff rate) needs to be defined based on a trade-off between measurement time and accuracy. As a general constraint, a sufficient number of oscillations must be detected so as to enable the calculation of the desired blood pressure-indicative values at a required accuracy. The measurement procedure as such is strongly dependent on the actual subject (patient), the type of the pressure cuff, the type of the pump, and the type and length of flow conduits (e.g., air tubes) that connect the pressure cuff and respective pressurizing units (e.g., pumps). Consequently, control of the inflation stage is crucial and of major importance for overall system accuracy and stability.

Document US 2014/257116 A1 describes an electronic blood pressure meter that includes an inflation control unit that controls a pump for outputting a fluid into a cuff so that a pressure in the cuff is increased at target inflation speed in accordance with a driving voltage. The electronic blood pressure meter further comprises a target changing unit that varies the target inflation speed during an inflation process so that the driving voltage measured during the inflation process stays within a voltage range corresponding to a range in which the pump is capable of output.

Another blood pressure meter is disclosed in US 2014/316290 A1, in which an adjustment unit adjusts a pressure in a cuff by controlling a piezoelectric pump that uses a piezoelectric vibrator to supply fluid to the cuff and a driving control unit that gradually changes a cuff pressure by performing driving control on the adjustment unit. The driving control unit carries out feedback control on the piezoelectric pump by means of a voltage based on a difference between the actual speed at which the cuff pressure is to be increased based on the cuff pressure inputted from a pressure detection unit and the current inflation speed target, so that the speed at which the cuff pressure is adjusted to reach the inflation speed target.

US 2012/323128 A1 relates to a device for inflating a cuff of a non-invasive blood pressure measurement apparatus, in which the cuff inflation speed is effectively adjusted on the basis of the measured actual pressure values of the cuff at the previous time points, the target pressure values of the cuff and the previous inflation speed parameters.

Published patent application JP 2006 129920 A describes a pressure control method and a pulse wave discrimination method of electronic sphygmomanometer in which cuff pressure is first rapidly increased to a prescribed pressure value and, after that, the rapid pressurization is shifted to slow pressurization. A feedback-controlled pressurization means controls that the slow pressurization speed becomes a prescribed target pressurization speed based on the difference between the computed average pressurization speed and the target pressurization speed.

Finally, US 2015/230718 A1 discloses a system for determining a blood pressure of a patient. The system includes a controller adapted to sense, measure, detect, monitor, calculate, and/or otherwise determine the blood pressure of the patient based on one or more hemodynamic parameters determined by a sensor. The controller may also control inflation of a cuff towards a target inflation pressure or to maintain the cuff at about the target inflation pressure.

Further, controlling a measurement system should be performed in a sensitive fashion so as to avoid levelling out the desired pressure oscillations that are indicative of the blood pressure. Nonetheless, controlling the measurement system should be performed in a sufficiently robust fashion so as to avoid undesired distortions and/or undesired termination of a measurement procedure. Further, different system parameters and/or measurement conditions shall be considered.

SUMMARY OF THE INVENTION

It is therefore an object of the present disclosure to seek for improved approaches to non-invasive blood pressure measurements which may be implemented in respective non-invasive methods and devices. Particularly, improved methods and devices for inflation stage-based non-invasive blood pressure measurement shall be presented.

More particularly, it is an object of the present disclosure to provide an improved inflation strategy for an inflation-based blood measurement procedure which addresses both accuracy and robustness issues. Further, the methods and devices in accordance with the present disclosure preferably enable the measurement of a subject's blood pressure within a short time. Preferably, discomfort for the monitored subject may be even further reduced without adversely affecting monitoring accuracy.

More particularly, it would be advantageous to provide a device and a corresponding method for oscillatory blood pressure monitoring which implement improvements in the signal control circuit so as to track and process the desired signal in a precise and sufficiently robust fashion.

These objects are solved by the blood pressure measurement device of claim 1, the blood pressure measurement method of claim 13 and the computer program of claim 15. Generally, the present disclosure attempts to provide a universally applicable control framework for the inflation stage of inflation-based blood pressure monitoring devices and methods.

More generally, in accordance with some aspects of the present disclosure, at least some drawbacks that are inherent to prior art inflation based blood pressure measurement approaches shall be addressed and mitigated. Further, at least in some aspects of the present disclosure, alternative approaches for signal processing in inflation based oscillatory blood pressure measurement devices and methods shall be presented.

In accordance with a first aspect of the present disclosure a blood pressure measurement device is presented, the device comprising a pressurizing unit(such as, for example, a pump), that is arranged to supply, via at least one supply line, a wearable cuff which is arranged to pressurize a measurement site of a subject of interest, a pressure detection unit that detects a cuff pressure $p_2$ in a direct or mediate fashion, a control section comprising a blood pressure determination unit arranged to calculate a blood pressure value based on the cuff pressure p2 detected by the pressure detection unit during inflation when the cuff pressure p2 is increased by the pressurizing unit in accordance with a defined pressurizing regime, a feed forward controller arranged to output a target inflation flow signal based on a desired value selected from the group consisting of desired inflation rate and desired pressure, and on a model of the pressurizing regime, and a feedback controller arranged to minimize the error between the desired value and an actual measured signal for the desired value, wherein the output of the feed forward controller and the output of the feedback controller are combined to drive the pressurizing unit.

The feed forward controller is arranged to output the target inflation flow signal based on the desired value and a model underlying the pressurizing regime. That is, the feed forward controller responds to the target inflation flow signal in a predefined way. independently of the reaction of the blood pressure monitoring system, and particularly the pressurizing regime. In that sense, the feed forward controller does not adjust its output in response to the actual measured inflation flow signal (i.e., the measured inflation rate or the measure pressure), because the feed forward control is not error-based.

On the other hand, the feedback controller has no knowledge of the system to be controlled and is arranged to react on the error signal between the target inflation flow signal and the actual measured signal in order to minimize said error. In other words, the feedback controller is not based on a model of the blood pressure monitoring system, and particularly of the pressurizing regime.

That is, while feedback control is error-based, feed forward control is in contrast model-based, in which an a priori knowledge of the process to be controlled is required.

This first aspect of the present disclosure is based on the insight that the feed forward controller may do the majority of the control work. Since a feed forward control as such is stable and fast and does not depend on the measured inflation rate (or pressure), fast and stable changes in inflation rate (or pressure) can be obtained without cancellation of the pressure oscillations of interest. Potentially remaining inflation rate (or pressure) deviations (compared to the desired inflation rate or pressure) that are detectable when monitoring the inflation processes may be compensated by the feedback controller to a great extent. However, the feed forward controller plays a dominant part in the control procedure such that the remaining workload for the feedback controller is relatively low. In one embodiment, the feedback controller may be adapted to a potential worst case scenario (in terms of cuff compliance) which results in well-balanced overall control performance (in terms of stability, accuracy, responsiveness and required measurement time).

The present disclosure focuses on the control of the inflation process. As indicated above, the inflation stage may be utilized for blood pressure measurement, provided that the inflation rate can be controlled. With respect to the blood pressure measurement as such, respective approaches and devices are well-known to the skilled person, refer also to the above-indicated US 2014/0309541 A1.

Preferably, the above-described device forms part of a blood pressure measurement system which also includes the cuff. In a more general context, the term cuff may be referred to as clamp unit. While most cuffs are arranged to be wrapped around a body limb, also clamp-like systems may be envisaged.

In accordance with at least some embodiments of the present disclosure, novel approaches to pressure rate control for inflation based NIBP measurement are presented. Accordingly, inflation control is stable, provides fast tracking and also does not level out cuff oscillations for a wide range of systems. In an embodiment, inflation speed can be related to the pulse rate (when the pulse rate is measured) such that an accurate inflation based NIBP measurement can be ensured for a wide variety of different patients in an as fast as possible way.

In one embodiment of the blood pressure measurement device, the feed forward controller is a continuous or quasi-continuous feed forward controller that is arranged to output a set inflation flow $I_{cuff}$ in a continuous or quasi-continuous fashion based on a desired inflation rate $dp_2/dt$ or desired cuff pressure $p_2$, and wherein the feedback controller is arranged to control remaining deviations between the desired and actual inflation rate $dp_2/dt$ or the desired and actual cuff pressure $p_2$ so as to complement the feed forward controller, and wherein the combined outputs of the feed forward and feedback controller are converted to an inflation flow set signal that is sent to the pressurizing unit.

In a further embodiment of the blood pressure measurement device, an update rate of both controllers is at least 10 Hz. Hence, control is basically continuous or quasi-continuous, such that the resulting cuff oscillation signal is not disturbed by any discontinuities due to the controller. Further, the feedback controller is a slow feedback controller, wherein the feedback controller gain(s) are chosen such that only low frequent deviations (compared to a setpoint) are corrected and oscillatory pressure signals which are indicative of blood pressure are not levelled out.

In yet a further embodiment of the blood pressure measurement device, the model underlying the pressurizing regime comprises a pneumatic flow circuit modelled on the basis of a reference pneumatic circuit involving the pressurizing unit and the cuff, when attached at the measurement site, and a supply line between the pressurizing unit and the cuff which corresponds to a modelled reference line resistance $R_{tube}$.

In an embodiment of the blood pressure measurement device, the pressurizing unit is a pump, and the feed forward flow is based on the compliance of the cuff that is attached to the measurement site, in accordance with the following equations:

$$(1) \qquad I_{pump}(t) \qquad = f(p_1, V_{pump})$$

$$(2) \qquad p_2(t) \qquad = 1/C_{cuff} \cdot \int_{t0}^{t} Icuff(\tau)d\tau + p2(t_0)$$

$$(3) \qquad \frac{dp_2}{dt} \qquad = 1/ C_{cuff} \cdot I_{cuff}$$

$$(4) \qquad p_1 \qquad = p_2 + I_{pump} \cdot R_{tube}$$

$$(5) \qquad I_{cuff} \qquad \approx I_{tube} \approx I_{pump},$$

wherein $p_1$ represents the pressure in the device before the tube, $p_2$ represents cuff pressure (behind the tube), $I_{cuff}$ represents an inflation flow generated by the pressurizing unit, $dp_2/dt$ represents an inflation rate of the cuff, $V_{pump}$ represents an input control signal (e.g., voltage signal or current/power signal) for the pump, $R_{tube}$ represents line resistance and $C_{cuff}$ represents cuff compliance. As used herein, the term compliance basically relates to the compressibility of the cuff when attached to the measurement site, hence also the compressibility of the respective body limb (e.g. upper arm portion) is reflected in the compliance value when the cuff is attached thereto and inflated. Note that cuff elastance ($E_{cuff}$) is the inverse of cuff compliance: $1/C_{cuff}$. Further, $t_0$ indicates initial conditions. As indicated above, cuff compliance may be referred to as combined cuff and limb compliance including the cuffs interaction with the measurement site when being pressurized. Typically, the cuff is attached to the upper arm of the patient.

In a further refinement, the control section is arranged to control at least one of the inflation rate $dp_2/dt$ or cuff pressure $p_2$ in a mediate fashion by adapting the inflation flow $I_{cuff}$ into the cuff, wherein the device pressure $p_1$ is measured during inflation, and wherein cuff compliance $C_{cuff}$ and line resistance $R_{tube}$ are determined at least once at the start of the measurement in accordance with the above equations. For instance, embodiments may be provided wherein only the device pressure $p_1$ is actually measured directly with a respective sensor while cuff pressure $p_2$ is not. However, a defined relation between device pressure $p_1$ and cuff pressure $p_2$ may be assumed as for example shown in equation (4). Consequently, as can be derived from the above equations, cuff pressure $p_2$ or cuff rate $dp_2/dt$ can be controlled by adapting the flow into the cuff, when cuff compliance $C_{cuff}$ and line resistance $R_{tube}$ are known and device pressure $p_1$ is measured.

By way of example, the line resistance $R_{tube}$ is determined by applying a defined flow step, and by determining a resulting device pressure step based on device pressure $p_1$ detection.

In a further exemplary refinement of the blood pressure measurement device, the cuff compliance $C_{cuff}$ at the measurement site is pressure dependent, wherein the cuff compliance $C_{cuff}$ is modelled as being at least one of linear or non-linear dependent on the cuff pressure rate $dp_2/dt$. For reasons of simplification, the pressure rate underlying the compliance detection may be the device pressure rate $dp_1/dt$ and not the cuff pressure rate as these rates are almost equal in case of a constant flow.

Alternatively, in this exemplary refinement, the cuff compliance $C_{cuff}$ is modelled as being at least one of linear or non-linear dependent on the cuff pressure $p_2$.

In a further exemplary embodiment, an initial cuff compliance $C_{cuff}$ at a certain initial pressure is determined based on an initial inflation flow value and an initial pressure rate value, wherein the cuff compliance $C_{cuff}$ during the remainder of the measurement is determined based on the initial cuff compliance, a continuous or quasi-continuous detection of device pressure $p_1$ and the compliance model.

In a further embodiment, the inflation flow value that is used to measure the compliance is the flow value that is based on a pump flow model.

By way of example, in another exemplary embodiment, a cuff-type dependent compliance estimation model is utilized, wherein the compliance model is based on the cuff type.

In a further exemplary embodiment of the blood pressure measurement device, the combined flow value from the feed forward and feedback controller are converted in a pump voltage, wherein the actual flow into the cuff is measured and wherein a flow feedback controller ensures that the resulting pump voltage $V_{pump}$ results in the requested flow. In accordance with this embodiment, a measurement of the actual flow is required which may be performed by a flow measurement unit.

In still another exemplary embodiment of the blood pressure measurement device, the combined flow value from the feed forward controller and the feedback controller is converted in a pump voltage $V_{pump}$ by a pump flow model that relates the inflation flow $I_{cuff}$ and device pressure $p_1$ to a pump voltage. The flow model basically linearizes the relation between the controller outputs and the actual flow output of the pump. The pump flow model also enables the determination of the inflation flow $I_{cuff}$ if the device pressure and voltage to the pump are known. The relation between flow, pressure and applied voltage is basically non-linear and pump-type dependent.

In accordance with another aspect of the present invention, a blood pressure measuring system is provided which implements a blood pressure measuring system in accordance with at least one embodiment as discussed herein, and a cuff or clamp unit connected thereto, wherein the cuff or clamp unit is arranged to pressurize a measurement site of a subject of interest for blood pressure related measurements.

In accordance with another aspect of the present invention, a blood pressure measurement method is presented, the method comprising the following steps:

- attaching a wearable cuff which is arranged to pressurize a measurement site of a subject of interest,
- operating a pressurizing unit (such as, for example, a pump) that is arranged to supply the cuff with a pressurized fluid via at least one supply line,
- detecting and monitoring a cuff pressure $p_2$ in a direct or mediate fashion,
- determining a blood pressure value based on the detected cuff pressure $p_2$ during inflation when the cuff pressure $p_2$ is increased by the pressurizing unit in accordance with a defined pressurizing regime, and
- controlling inflation of the cuff, comprising:

    - setting a required inflation flow $I_{cuff}$ by means of a feed forward controller, including defining a target inflation flow signal based on a desired value selected from the group consisting of desired inflation rate and desired pressure, and on a model of the pressurizing regime, and
    - controlling the desired value by means of a feedback controller, including minimizing the error between the desired value and an actual measured signal for the desired value,

wherein the output of the feed forward controller and the output of the feedback controller are combined to drive the pressurizing unit.

In accordance with this method, a blood pressure measurement system may be adequately driven in a fast but also precise manner. Further, blood pressure measurement procedures may be accomplished in a more rapid fashion.

In accordance with an exemplary refinement of the above method, there are provided the steps of:

- enabling, particularly initiating, the pressurizing unit by a defined flow step,
- detecting a pressure step at the pressurizing unit or the cuff in response to the applied flow step,
- calculating line resistance $R_{tube}$ based on the pressure step and the applied flow step,
- determining an initial cuff compliance $C_{cuff}$ value based on a defined flow value and a pressure rate value,
- identifying an actual cuff type based on the detected cuff compliance $C_{cuff}$ value,
- tracking the cuff compliance $C_{cuff}$ based on a compliance estimation model and ongoing cuff pressure detection,
- inflating the cuff under control of the feed forward controller, wherein a desired inflation rate $dp_2/dt$ or pressure $p_2$ is set,
- applying feedback control by the feedback controller to adapt the inflation flow $I_{cuff}$ based on detected deviations between the desired inflation rate $dp_2/dt$ (or pressure $p_2$) and an actual inflation rate (or pressure), and
- calculating blood pressure based on the observed cuff pressure $p_2$ at the inflation stage.

In yet another aspect of the present invention there is provided a computer program which comprises program code means for causing a computer or a processor included in the blood pressure measurement devices as discussed herein to perform the steps of the methods as discussed herein when said computer program is carried out on that computer or that processor.

The program code (or: logic) can be encoded in one or more non-transitory, tangible media for execution by a computing machine, such as a computer. In some exemplary embodiments, the program code may be downloaded over a network to a persistent memory unit or storage from another device or data processing system through computer readable signal media for use within the system. For instance, program code stored in a computer readable memory unit or storage medium in a server data processing system may be downloaded over a network from the server to the system. The data processing device providing program code may be a server computer, a client computer, or some other device capable of storing and transmitting program code.

As used herein, the term "computer" may stand for a large variety of processing devices. In other words, also mobile devices having a considerable computing capacity can be referred to as computing devices, even though they provide less processing power resources than standard "computers". Needless to say, such a "computer" can be part of a medical device and/or system. Furthermore, the term "computer" may also refer to a distributed computing device which may involve or make use of computing capacity provided in a cloud environment. The term "computer" may also relate to medical technology devices, fitness equipment devices, and monitoring devices in general, that are capable of processing data.

Preferred embodiments of the disclosure are defined in the dependent claims. It should be understood that the claimed method and the claimed computer program can have similar preferred embodiments as the claimed system and as defined in the dependent system claims.

BRIEF DESCRIPTION OF THE DRAWINGS

These and other aspects of the invention will be apparent from and elucidated with reference to the embodiments described hereinafter. In the following drawings

Fig. 1 shows a simplified schematic illustration of a blood pressure monitoring system including a blood pressure monitoring device;

Fig. 2 illustrates an exemplary measurement environment for a blood pressure monitoring system;

Fig. 3 shows a simplified schematic representation of a circuit based on which a model for operating a blood pressure monitoring system may be established;

Fig. 4 shows a chart illustrating exemplary pressure over time curves including a device pressure curve and a corresponding cuff pressure curve;

Fig. 5 shows a chart illustrating exemplary compliance over pressure curves for different cuff types;

Fig. 6 shows a chart illustrating exemplary pump flow rates over pressure curves for different driving voltage values;

Fig. 7 shows an exemplary simplified schematic block illustration of the function of a linearizing unit which may be implemented in a control section of a blood pressure monitoring device;

Fig. 8 shows several synchronized charts illustrating exemplary curves of relevant signals for inflation-based blood pressure monitoring;

Fig. 9 shows an illustrative block diagram representing several steps of an embodiment of a blood pressure monitoring method in accordance with the present disclosure; and

Fig. 10 shows an illustrative block diagram representing several steps of another embodiment of a blood pressure monitoring method in accordance with the present disclosure.

DETAILED DESCRIPTION OF THE INVENTION

In the following, main aspects and insights of the invention will be illustrated and further elucidated with reference to exemplary embodiments of inflation-based NIBP methods and devices. It should be noted that the following exemplary embodiments and descriptions shall be not interpreted in a limiting sense. Rather, the skilled person may readily transfer and broaden the respective specific embodiments and the components as well as process steps disclosed therein so as to arrive at the general concept of the present disclosure.

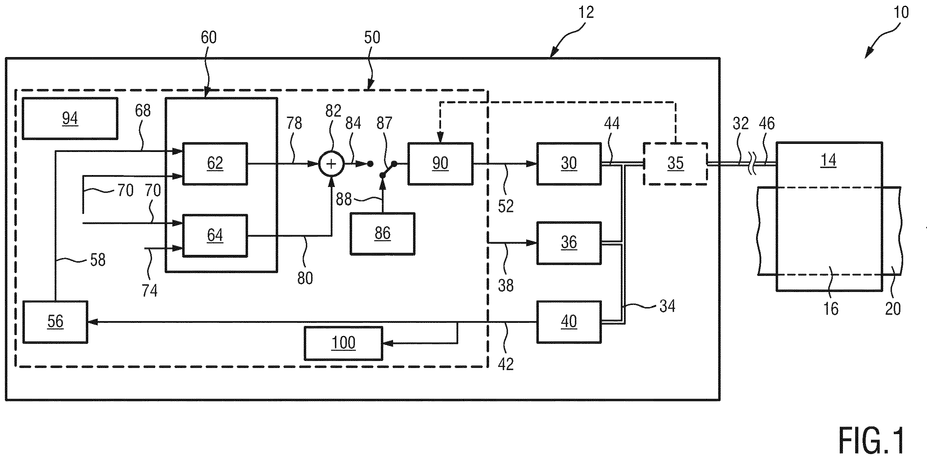

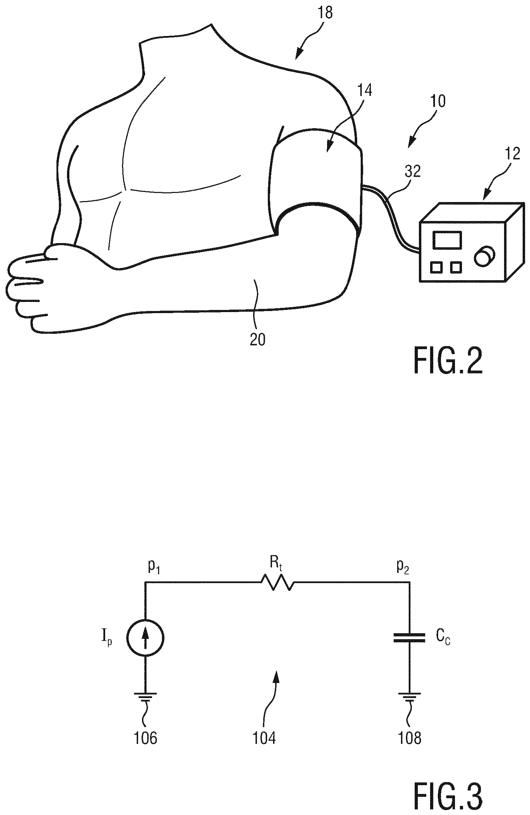

Reference is made to Fig. 1 illustrating a simplified schematic block representation of a blood pressure monitoring system 10 that implements a blood pressure monitoring device 12 in accordance with the present disclosure. The system 10 further comprises a cuff or clamp unit 14 which may be attached to a measurement site 16 of a subject of interest 18 (exemplarily illustrated in Fig. 2), particularly at a body limb 20 thereof. Typically, the cuff 14 may be attached to the upper arm of a subject.

Again, reference is made to Fig. 1. With respect to the general structure and to general features of exemplary conventional inflation-based NIBP devices, reference is made to US 2014/0309541 A1 disclosing an oscillometric blood pressure measurement device which may be operated to measure the blood pressure at the inflation stage of a cuff.

The device 10 comprises a pressurizing unit 30 which may comprise at least one pump or pump unit. The pressurizing unit 30 may be driven so as to enable a defined inflation regime of the cuff 14 which allows detecting the blood pressure already in the inflation stage. The pressurizing unit 30 is connected to or connectable to the cuff 14 via a line arrangement involving respective tubes, particularly via at least one supply line 32.

Typically, the pressurizing unit 30 is arranged to output compressed air via the supply line 32 to the cuff 14 so as to inflate the cuff 14 and to pressurize the measurement site 16 at the limb 20 of a subject. Further (internal and external) flow lines 34 may be provided so as to connect the pressurizing unit 30 to an exhaust (or relief) valve 36, for instance, and to at least one pressure determining unit 40. The exhaust valve 36 is arranged to deflate the cuff 14 in a defined fashion. The fluid lines are shown for illustrative purposes in Fig. 1 do not necessarily have to correspond to a particular physical line-run arrangement.

The at least one determining unit 40 comprises a pressure sensor and is arranged to output a pressure signal 42 related to the device pressure 44 (and implicitly to cuff pressure 46). On a long-term basis, when no pressure changes occur, device pressure $p_1$ and cuff pressure $p_2$ basically correspond to one another. However, to control inflation of the cuff 14 in the desired accurate fashion, at least temporal deviations between device pressure $p_1$ and cuff pressure $p_2$ have to be considered. Exemplary measurement points for device pressure $p_1$ and cuff pressure $p_2$ are indicated in Fig. 1 by reference numerals 44 (device pressure $p_1$) and 46 (cuff pressure $p_2$), respectively.

Further, in some exemplary systems 10 within the general context of the present disclosure, an interface between the cuff 14 and the device 10 may be provided such that both entities may be separated. Further, as will be discussed herein below, different types of pressurizing units 30 and/or cuffs 14 may be combined which may also involve differently shaped supply lines 32.

The system 10 further comprises a control section 50 for controlling the inflation of the cuff 14 in the desired fashion. This is mainly accomplished by operating the pressurizing unit 30 accordingly so as to achieve a defined target inflation rate (or target pressure) at the cuff 14. To this end, a rate calculator 56 may be provided which is arranged to calculate a present value, such as a current inflation rate ($dp_1/dt$, see further below). As an input value, the pressure signal 42 may be supplied to the rate calculator 56. The calculated signal, for instance an inflation rate representing signal 58, is forwarded to an inflation controller 60 which implements a feedback controller 62 and a feed forward controller 64. The inflation rate 58 calculated by the rate calculator 56 (or the pressure 42) serves as an input feedback signal 68 for the feedback controller 62. Further, a target signal 70 (inflation rate target or pressure target) may be supplied to the feedback controller 62 which enables respective tracking and adjusting.

The same target signal 70 may be used for the feed forward controller 64. Additional parameters 74 may be supplied to the feedforward controller that basically represent the underlying feed forward control regime. Particularly, these additional parameters 74 consist of a cuff compliance value and possibly a tube resistance value. In accordance with exemplary embodiments of the present disclosure, the feed forward controller 64 plays a dominant part in the control process. Accordingly, the feedback controller 62 is provided for compensating remaining signal deviations which basically cannot be predicted by the feed forward model.

Respective output control signals of the feedback controller 62 and the feed forward controller 64 are signals 78 and 80, namely an (output) feedback signal 78 and an (output) feed forward signal 80, which basically define a required flow signal for the pressurizing unit 30. The feedback signal 78 and the feed forward signal 80 are combined, at reference numeral 82, and jointly form a combined required flow signal 84 which eventually may be supplied to the pressurizing unit 30.

Further, a switch 87 may be provided that selects either the combined controlled flow signal 84 or an open loop flow 88. The main application for this can be found in an initial stage of a measurement procedure when system settings parameters need to be determined. The position of the switch and the value of the open loop flow may be set by a state processor 94, which enables / disables system settings depending on the state in which the blood pressure device operates. For example, during the determination of the resistance $R_{tube}$ and/or the compliance $C_{cuff}$, a defined flow $I_{pump}$ has to be applied, and not a flow that is based on a defined target inflation rate $dp_2/dt$. The switch 87 may be operated accordingly.

Further, so as to facilitate operating and controlling the pressurizing unit 30, a flow to pump voltage converter 90 may be provided which translates the requested flow 84 to an overall driving signal 52 for the pressurizing unit 30. The driving signal 52 may be for instance a voltage signal for driving the pressurizing unit 30.

In accordance with at least some embodiments of the present disclosure, the flow to pump voltage convertor consists of a flow feedback control loop that minimizes the difference between the desired flow 84 and an actual flow 39. In order to measure the actual flow 39, a flow signal measurement sensor 35 is placed in the inflation line.

In another embodiment the flow to voltage converter 90 consists of a linearizing unit that cancels the transfer function of the pump and ensures in this way that a requested flow is generated by the pump (by making the requested flow - to - actual flow relation linear). The linearizing unit basically performs an inverse pump function. Where the pump 30 translates a voltage into a flow value in a nonlinear fashion, the linearizer translates a desired flow into a pump voltage such that the overall transfer function is linear. By linearization of the pressurizing unit (i.e. the pump), a certain flow value can be assured at a certain controller output (i.e. without linearization, a pump input of a certain voltage level might not result in a twice as high output flow as an input voltage which is half a large). A linearizing unit before the pump can ensure that a control signal (flow) is converted to a voltage such that a twice as high control signal (flow) results in a twice as high output flow. This linear behavior is beneficial for the controllers, which can be used in their optimal regime.

When using the linearizing unit, the flow sensor 35 can be omitted.

In another embodiment, the flow feedback controller and the linearizer are implemented in a combined fashion which may further improve the inflation control performance. Eventually, the device 10 comprises a blood pressure detection unit 100 which is arranged to process and calculate the desired signal of interest during in inflation process when the cuff 14 is attached to the measurement site 16.

Generally, processing units and control units referred to herein (for instance the exemplary entities 50, 56, 60, 62, 64 and 90 of Fig. 1) may be implemented in hardware and/or software. Further, features and functions of particular ones of these entities may be embodied and provided also by other control entities. Exemplary embodiments as discussed herein are primarily provided for illustrative purposes, and not for delimiting the scope of the disclosure. Consequently, exemplary features and functions described herein may be provided by a common processing entity and/or by distributed elements.

Having illustrated a general layout of a system and a device in accordance with the current invention, the above-discussed model-based approach will be further illustrated and explained hereinafter.

Relevant aspects of the present disclosure are based on the main idea to model a corresponding inflation based NIBP system in basic elements, to identify the values of these elements initially in a measurement (or factory calibration), to linearize the system and to apply specific feed forward control based on the identified elements. It is further assumed that, when the system elements are known, a large part of inflation control can be done by feed forward control as the characteristics of the system are known and described in the model.

Reference is made to Fig. 3 illustrating an exemplary model for blood pressure measurement systems. It goes without saying that further alternative models may be utilized, some of which may even provide a more detailed representation of the measurement device and system. In Fig 3, the NIBP system components are described in lumped elements. A pump is represented by a flow (or pressure) source. A tube or fluid line arrangement is represented by a (line) resistance. Further, the cuff in its attached state, when attached to the measurement site, is represented by a compliance. In total, the exemplary model is designated by reference numeral 104. Reference numerals 106, 108 indicate atmospheric pressure.

Consequently, a model of the NIBP system 10 may be established with the following few main system elements. A pump (or pressurizing unit) is provided and arranged to inflate the cuff and build up cuff pressure. The pump is modelled by a flow source (or a pressure source). In the present example, the relevant quantity is the outputted flow rate $I_{pump}$. A flow line arrangement or, put simply, a tube is provided that connects the pump to a cuff. In the present example, the relevant quantity is (line) resistance $R_{tube}$. A cuff is provided that is arranged to cooperate with a measurement site (at a body limb, e.g. an arm portion) to increases the pressure applied thereto. Pressure increase may occur as a function of cuff volume. In the present example, the relevant quantity is compliance $C_{cuff}$. Note that cuff elastance ($E_{cuff}$) is the inverse of cuff compliance, $1/C_{cuff}$.

Given the above model 104, the modeled system 10 may be described and analyzed based on the following equations:

The relations between cuff pressure $p_2$ with initial condition $p_2(t0)$, device pressure $p_1$, pump flow $I_{cuff}$ and (cuff) inflation rate $dp_2/dt$ can be described as follows:

$$(1) \qquad I_{pump}(t) \qquad = f(p_1, V_{pump})$$

$$(2) \qquad p_2(t) \qquad = 1/C_{cuff} \cdot \int_{t0}^{t} Icuff(\tau)d\tau + p2(t_0)$$

$$(3) \qquad \frac{dp_2}{dt} \qquad = 1/ C_{cuff} \cdot I_{cuff}$$

$$(4) \qquad p_1 \qquad = p_2 + I_{pump} \cdot R_{tube}$$

$$(5) \qquad I_{cuff} \qquad \approx I_{tube} \approx I_{pump}.$$

It is worth noting that in most NIBP systems the pressure $p_1$ is actually measured with a sensor (see unit 40 at Fig. 1), while $p_2$ is typically not measured in a direct fashion. However, a relation between $p_1$ and $p_2$ may be assumed so that $p_2$ can be detected in a mediate fashion. The above equations indicate that cuff pressure $p_2$ or cuff inflation rate $dp_2/dt$ may be eventually controlled by adapting the pump flow $I_{cuff}$ into the cuff, when compliance $C_{cuff}$ and resistance

$R_{tube}$ are known and device pressure $p_1$ is measured.

In control theory terms, the system of equations (1) to (5) may be processed and rearranged to represent a system pole at -1/ ($R_{tube} \cdot C_{cuff}$). If a controller would be used that only incorporates feedback, the gains of this controller (e.g. a combination of proportional integral and derivative control, PID) have to be chosen such that the system tracks a setpoint quickly and is stable for different $R_{tube}$ and $C_{cuff}$ values, as a blood pressure measurement system should be able to work with different cuffs and tubes. As the range of cuff compliances and tube resistances is large, certain feedback control gain setting(s) could result in a fast and stable system for a certain $R_{tube}$ and $C_{cuff}$, where it for another combination of $R_{tube}$ and $C_{cuff}$ could result in unstable behavior or slow reaching of target values. Further, also variations over time (cuff compliance is not constant over pressure) are present. Besides these issues, the pressure oscillations should not be levelled out, requiring a sufficiently slow control. On the one side high control gains are required that would result in fast reaction of the system, where on the other low control gains are needed in order to have a stable system for different cuffs and tubes and to not cancel out pressure oscillations.

As a consequence, as discussed herein, a control strategy based on combined feed forward and feedback control is a beneficial approach for practical use.

In accordance with at least some embodiments of the present disclosure, the following insights may apply. Generally, continuous or quasi-continuous (i.e. sampling and processing rate at least 10 Hz) feed-forward control may be applied under consideration of the above-presented equations (1) to (5). Hence, the required flow may be calculated based on a desired pressure rate or pressure profile. This can be achieved without the need of providing a feedback loop for the desired rate or pressure. In an exemplary embodiment the flow is assumed to be dependent on the desired inflation rate:

$$Required\ Flow\ [mL/s] = Cuff\ Compliance\ [mL/mmHg] \cdot Desired\ Inflation\ Rate\ [mmHg/s]$$

An advantage of the feed forward control is that it is inherently stable. Another advantage of the feed forward method is that cuff oscillations are not controlled out as the pump output is not based on the actual pressure or rate, but only on the value of the compliance and the set point rate or pressure. The feed forward method basically requires that cuff compliance (and possible resistance) are known or detectable or can be at least approximately determined, and that a required flow can be made by the pump.

As in practice the measurement of $R_{tube}$, $C_{cuff}$ will not necessarily be perfect and also the (rather simplified) model will not be perfect (e.g. due to simplifications, parasitic elements, etc.), the resulting rate or pressure will not perfectly reflect the desired rate/pressure. However, the major part (e.g. 80 - 90%) of the desired pressure or rate profile can be achieved. The remaining 10% -20% part may be compensated by additional supplemental (auxiliary) controls.

For instance, a feedback controller may be provided which is arranged for stable control in a worst case scenario (i.e. a worst case $R_{tube}$, $C_{cuff}$ combination). This may be achieved for example by a combination of P, I and D, as indicated above. The operation of this controller may be considerably slow as it is arranged for the worst case scenario. However, control speed is already ensured by the feed forward controller, resulting in total in a quick, almost correct feed forward rate or pressure, which may be slowly refined and updated to obtain target values.

Further, a feedback controller comprising adjustable control gains (e.g. a combination of P, I and D) may be provided, wherein adjustment of the control gains may be based on the actual RC combination such that stability and tracking requirements are met for the specific system. For example, control gains may be selected to be proportional to the $R_{tube}$ and $C_{cuff}$ combination (e.g. $G = G_{fixed} + k_G \cdot R_{tube} \cdot C_{cuff}$). This method does not implicitly ensure that cuff oscillations are not controlled out, which should be taken care for by conservatively choosing the proportionality factor.

In an exemplary embodiment, a feedback controller implementing integral control (I-control) only is utilized for the feedback part wherein the setting of the integral term is based on worst case $R_{tube}$ and $C_{cuff}$ parameters. In accordance with this exemplary embodiment, P-control and D-control is avoided as these terms might adversely affect signal oscillations that are representative of the desired blood pressure. Controlling out or leveling off these oscillations should be avoided.

For applying the above-described control strategies, the $R_{tube}$ and $C_{cuff}$ parameters need to be known. They can be identified in accordance with the following exemplary approaches.

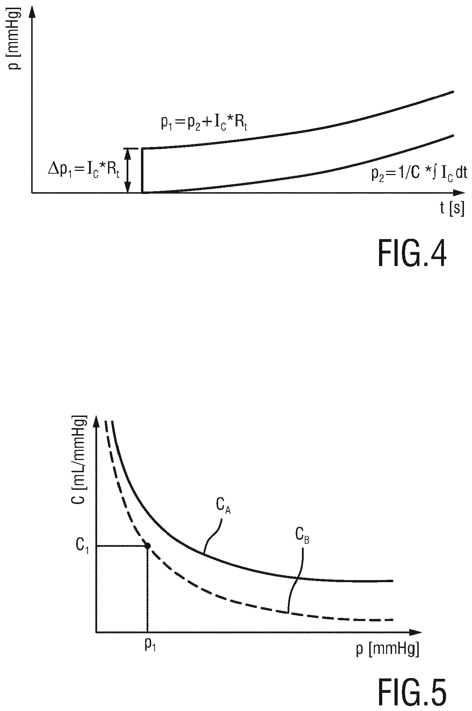

The tube resistance $R_{tube}$ may be identified by applying a known flow step to the system (refer also to Fig. 4). The cuff pressure ($p_2$, normally not available in a standard NIBP system) increases slowly by the integral of flow. The device pressure p1 increases at the same rate, however at the moment of a flow step, a step in pressure can be observed. This step is basically proportional to the flow through the resistance ($\Delta p_1 = I_{cuff} \cdot R_{tube}$). Therefore, the tube (or line) resistance can be calculated as follows: $R_{tube} = \Delta p_1 / I_{cuff}$. $\Delta p_1$ can be determined by the pressure difference between the pressure just before the flow step is applied and the pressure just after the flow step.

It is worth noting that the flow step can be made in several ways and at several time points. Further, the flow can be measured (with a sensor) or be estimated (with a model). In one embodiment, the flow step is made by requesting an open loop flow 88 that is translated to a pump voltage by the flow to voltage convertor 90. In another embodiment, the flow step is made by opening the exhaust valve 36 for a certain amount of time. In one embodiment, the flow may be modeled as a function of the pump voltage $V_{pump}$ and pressure $p_1$. The flow step as such may be applied at the start of the NIBP measurement when the cuff is in a deflated state and inflation starts, preferably on a one-time basis. Further, the flow step as such may be applied at the end of the inflation as a negative step to stop the flow, preferably on a one-time basis. Further, in the alternative, the flow step may be applied repetitively. Consequently, average tube resistance values for a more accurate measurement may be determined. It is worth noting that $R_{tube}$ is approximately constant over pressure. Further besides for the $R_{tube}$, $C_{cuff}$-combination, also $R_{tube}$ basically needs to be known for determination of the cuff pressure $p_2$, as for instance systolic blood pressure and diastolic blood pressure need to be determined as function of cuff-pressure $p_2$, and not as function of device pressure $p_1$.

Cuff (and arm) compliance $C_{cuff}$ can be estimated by dividing the flow by the pressure rate, see equation (3) above. For the sake of simplification, the pressure rate at the pump $p_1$ can be used instead of the (basically not available) cuff pressure $p_2$ at a stage when the flow is approximately constant which may be practically anytime but at the step of enabling/disabling the flow. Further, a measured flow or modeled flow can be used in order to determine compliance. In case of flow measurement, a respective flow measurement sensor 35 is required.

Cuff (and arm) compliance $C_{cuff}$ may be non-constant over pressure. For example an inverse linear or inverse logarithmic relation with pressure may occur. In an exemplary embodiment, the compliance value is updated regularly by doing repetitive measurements during inflation.

In another exemplary embodiment, (a) model(s) for the cuff (and arm) compliance $C_{cuff}$ is (are) used, refer also to Fig. 5. In accordance with the model, the compliance $C_{cuff}$ is dependent on cuff pressure $p_2$. A one-time compliance measurement may be performed that determines what compliance is currently in the system (e.g. CA or CB as indicated in Fig 5). The compliance value can also be used to identify the cuff type. During inflation, the current compliance value $C_{cuff}$ is updated as function of measured pressure $p_1$, the one-time measured compliance and the model.

Several embodiments can be implemented. Individual curves can be modeled for separate cuffs. In another exemplary embodiment, a single parameter function describes the relation between pressure and compliance for all cuffs. Consequently, individual compliance models are not required, but a continuous set of models may be provided which may account for small individual compliance differences due to e.g. different wrapping conditions of a particular cuff (or cuff type).

An exemplary cuff compliance model may be established as follows:

$$Compliance \ [mL/mmHg] = 1/(K_{Elastance}\,[1/mL] \cdot pressure \ [mmHg]),$$

wherein

$$K_{Elastance}\ [1/mL] = Rate \ [mmHg/s] / (Flow \ [mL/s] \cdot \ pressure \ [mmHg])$$

A single-time measurement of pressure, pressure rate and compliance can determine the constant $K_{Elastance}$. In the remainder of the measurement the identified model can be used, as $K_{Elastance}$ is now determined. As the pressure is measured basically continuously, a compliance value can be calculated continuously from the time point that the compliance was identified. A benefit of the model approach is that the measurement of the compliance is done only one-time, thereby not having any pressure oscillations occurring in the compliance value.

In order to have a properly functioning system, the requested flow (basically corresponding to $I_{pump}$, see equation (5) above) by the controller has to be delivered by the pump with a certain accuracy. Different implementations can be used to ensure this.

In one embodiment, the flow $I_{cuff}$ may be measured with a flow sensor, placed somewhere in the system, preferably just after the pump. A flow feedback controller is implemented that minimizes the difference between the required (set point) flow and the measured flow. In one embodiment, the feedback controller is a PID controller.

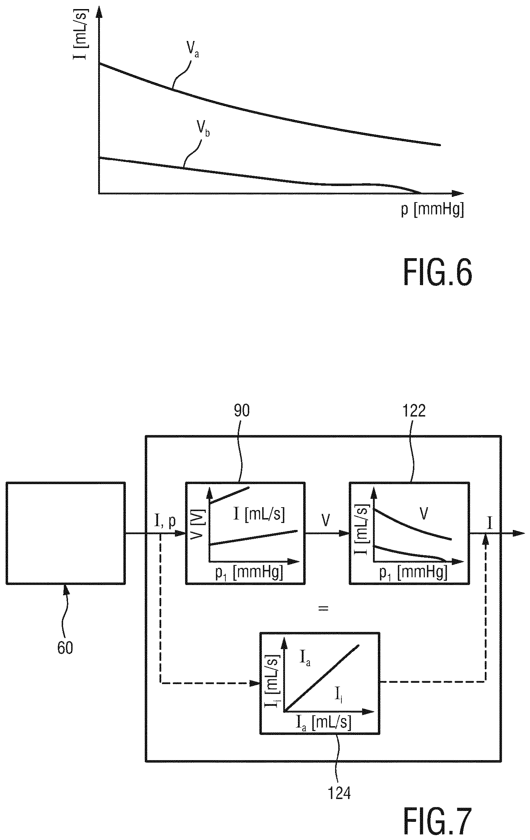

In another embodiment, a certain flow $I_{cuff}$ from the pump may be guaranteed by modeling the flow as a function of applied voltage $V_{pump}$ and pressure at the pump $p_1$ and inserting a linearizer block that cancels out this pump transfer function, refer also to Fig. 6, i.e. flow = $f$(*Voltage, pressure*) and Fig 7. In Fig. 6, flow is indicated by the symbol I referring to flow rate. Further, chart a may represent a voltage a, whereas chart b may represent a voltage b. Generally, flow is indicated by the letter I in this disclosure. The pump flow model could be determined a single time for a certain pump type. For an enhanced flow model, each individual pump function could be identified (e.g. via factory calibration). Different model types identifying different pump types may be used, e.g. a lookup table, or a polynomial model, where appropriate.

As indicated in the charts of Fig. 6, for most pump types, the relation between flow, pressure and applied (driving) voltage is basically non-constant or non-linear, where a certain flow value from the pump can only be guaranteed if the pump outputs a flow that is proportional to the controller output. By inserting a linearizing unit (see figure 7), the pump non-linearity may be compensated for. By inserting the linearizing unit, the output of the controllers correspond to a flow value, rather than to a voltage value for driving the pump, as the linearizing unit transforms the requested flow to a voltage. Fig. 7 illustrates an exemplary embodiment of a linearizing unit 90 which is coupled to an inflation controller 60, and to a pressurizing unit 30 (not shown in Fig. 7), refer also to Fig. 1. The linearizing unit 90 is arranged to adjust the control signals so as to establish a basically linear relationship between input values provided by the inflation controller 60 and a resulting output flow of the pump or pressurizing unit 30.

System linearization is beneficial for both feed forward control and feedback control. For both controllers it ensures that the required flow is actually made by the pump. For feed forward control, a nonlinearity would results in a mismatch between required (target) flow output and actual flow output. Feedback control theory is only valid for linear actuators. Nonlinearities may therefore result in far from optimal control performance. Hence, the output of the pressurizing unit 30, due to the insertion of the linearizing unit 90, is basically linear, i.e. the outputted flow is basically proportional to the output of the controller.

Processing operation of the linearizing unit 90 resulting in a linear transfer function is illustrated by schematic blocks 90, 122 and 124 in Fig. 7, where block 90 is the linearizer, block 122 represents the pump transfer function and 124 is the transfer function of the linearizer and pump combined. Most pumps have a basically non-linear flow/ pressure / input voltage relation (relation between $I_{pump}$, $p_2$, and $V_{pump}$), refer to blocks 90 and 122 in Fig. 7. The non-linear transfer function(s) can be leveled by interposing the linearizing unit 90 between the inflation controller 60 and the actual pressurizing unit 30. The linearizing unit 90 is arranged to exhibit and to apply the inverse relation of the pump function to linearize the pump nonlinearity. By applying the linearizing unit 90, the flow output of the pump is linear to the input of the linearizing unit 90, refer to block 124 in Fig. 7 (i.e. the total transfer function = constant, resulting in a linear requested flow - actual flow relation). Therefore, the inflation controller 60 now outputs, so-to-say, a direct target flow value instead of a mere driving voltage value to the pressurizing unit 30. The resulting transfer function is illustrated by the block 124 which is shown for illustrative purposes as a result of the operation of the linearizing unit 90 in accordance with the (inverse) transfer function as indicated by Fig. 7.

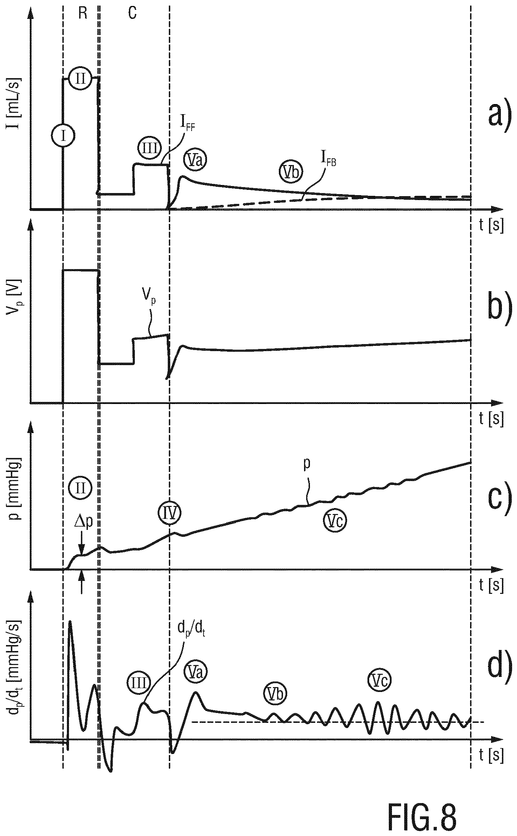

With particular reference to Fig. 1 and to Fig. 8, an exemplary embodiment and the operation thereof will be further detailed. In accordance with this embodiment, several of the above-discussed elements and aspects are implemented in a single inflation based blood pressure measurement device and a corresponding method. This embodiment includes a pump flow model which is incorporated in a linearizing unit which is established as a function of (device) pressure $p_1$ and pump voltage $V_{pump}$ which may involve one-time factory calibration. Consequently, flow $I_{cuff}$ is measured as a function of the measured pressure $p_1$ and the requested pump voltage $V_{pump}$. It is worth noting that in this embodiment the flow control as such is basically open loop, i.e. there is no direct feedback loop that measures the exact flow and regulates it to a precise value. The variable to be controlled is inflation rate. No direct control of pressure $p_1$ is performed. As feedback controller, an integral-only feedback controller (I-controller) is used, with a gain setting that is selected to be basically stable for any cuff and tube combination that may be envisaged (including worst case scenario).

An exemplary measurement sequence is illustrated in Fig. 8. Fig. 8 shows several charts illustrating flow $I_{cuff}$ (indicated by the symbol I), Pump voltage $V_{pump}$ (indicated by the symbol $V_p$), pressure ($p_1$ or $p_2$), and inflation rate ($dp_1/dt$ or $dp_2/dt$). Flow is plotted over time in in section a). Pump voltage is plotted over time in section b). Pressure is plotted over time in section c). Inflation rate is plotted over time in in section d). Accordingly, section a) illustrates a requested flow output by the (combined) inflation controller 60, refer to Fig. 1. Section b) illustrates an actual pump voltage. Section c) illustrates a measured cuff pressure. Section d) illustrates a measured inflation rate.

Several characteristic stages and/or events in the measurement procedure are indicated in the charts of Fig. 8 by I, II, III, IV, and V (including Va, Vb, and Vc).

In accordance with the exemplary procedure, the following sequence may apply:

I. The pump 30 is enabled by a step in flow. A high flow value is requested.
II. The resulting resistance ($R_{tube}$) is measured based on the induced step in pressure that is observed and the flow value $I_{cuff}$ that was requested.
III. When the pressure reaches a certain value, a dedicated flow profile is applied to measure one point of the (pressure dependent) cuff compliance $C_{cuff}$. This is achieved by dividing the requested flow value $I_{cuff}$ by the measured inflation rate $dp_2/dt$.
IV. The measured compliance point $C_{cuff}$ identifies which cuff compliance 14 (and which cuff type) is actually used in the system 10. For the remainder of the measurement sequence, the pressure $p_1$ is measured and the compliance model (which is dependent on pressure $p_1$) is used to obtain the (pressure dependent) compliance $C_{cuff}$ for the identified cuff type.
V. Inflation of the cuff 14 is started and a desired rate $dp_2/dt$ is requested. The inflation controller 60, particularly the feedforward controller 64 thereof, controls the inflation rate $dp_2/dt$. The desired rate $dp_2/dt$ may be a fixed rate, or may be based on the pulse rate of the patient 18 that is measured. The desired target rate $dp_2/dt$ is indicated in chart d) by a broken line.

   Va. The requested flow is based on the values of the compliance $C_{cuff}$ (and the resistance $R_{tube}$). As this is a feed forward value only, there may be a remaining small error compared to the desired rate.
   Vb. A slow integral feedback controller 62 is provided which integrates the error between the desired inflation rate and actual inflation rate $dp_2/dt$, and slowly corrects the inflation rate to approach the target value.
   Vc. As the I-controller 62 is relatively slow, it does not cancel out the oscillations in the pressure $p_2$ (and the inflation rate $dp_2/dt$).

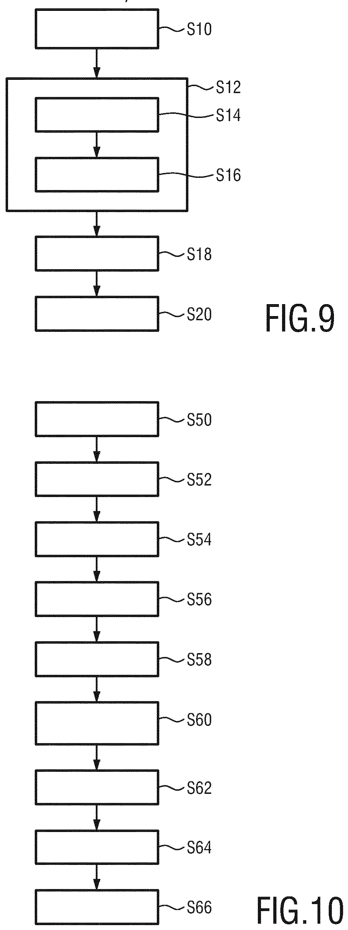

Needless to say, the above exemplary measurement sequence shall not be construed in a limiting sense.
Reference is made to Fig. 9 illustrating a simplified block diagram exemplifying an embodiment of a method in accordance with the present disclosure. Initially, in a step S10, a blood pressure monitoring system within the general context of the present disclosure is provided. Further, a wearable cuff is attached to a measurement site of a subject of interest.
A subsequent (generic) step S12 is related to the operation of a pressurizing unit, particularly a pump, of the system in accordance with at least some of the control approaches as discussed herein. Generally, the pressurizing unit is arranged to supply the cuff with a pressurized fluid via at least one supply line. Particularly, step S12 may involve controlling inflation of the cuff.
Step S12 exemplarily involves (sub)steps S14 und S16. Step S14 relates to feed forward control using a feed forward controller. Step S16 relates to supplementary feedback control using a feedback controller, for instance an I-controller. An I-controller implements an integral section. Further types of feedback controllers may be envisaged.
Inflation control may involve setting a required inflation flow $I_{cuff}$ by means of a feed forward controller, step S14. Supplemental feedback control of an actual inflation flow may be performed by means of a feedback controller, step S16. In the step S12, the output of the feed forward controller and the output of the feedback controller are basically combined to drive the pressurizing unit. Preferably, the model based on which the feed forward controller is operated is sufficiently accurate, particularly in such a way that auxiliary feedback control is only used for a small remaining signal deviation fraction.
A further step S18 relates to the detection of a cuff pressure $p_2$ in a direct or mediate fashion. Further, the cuff pressure $p_2$ may be monitored over time in a continuous or quasi-continuous fashion. A cuff pressure signal obtained in the step S18 also may be used as a (feedback) input signal for the feedback control performed in the step S16.
At least some of the steps S12, S14, S16 and S20 may be carried out in a basically parallel or synchronous fashion. Further, the steps of accordance with this method are carried out in repetitive (constant or quasi-constant) fashion.
Further, in a step S20, a blood pressure value may be processed and calculated based on the detected cuff pressure $p_2$ during inflation when the cuff pressure $p_2$ is increased by the pressurizing unit in accordance with a defined pressurizing regime.
Further reference is made to Fig. 10 illustrating a simplified block diagram exemplifying an embodiment of a method in accordance with the present disclosure. The method as illustrated in Fig. 10 may be regarded as an exemplary more detailed embodiment of the general method as presented in Fig. 9. Fig. 10 describes a complete measurement cycle. Fig. 10 focuses on the operation of the system and the device. Therefore, cuff attachment, system initialization, etc., is not described in Fig. 10. Further, Fig. 10 illustrates an exemplary method of operating a device 12 as illustrated in Fig. 1, whereas signal charts of a corresponding measurement sequence are illustrated in Fig. 8.
At a step S50, the pump is enabled by a step. Pump operation is initiated. For instance, a high flow value $I_{cuff}$ is requested. At a subsequent step S52, detection of the device pressure $p_1$ which may basically correspond to the cuff pressure $p_2$ is initiated. Steps S50 and S52 may basically start at about the same time. At a further step S54, the resistance $R_{tube}$ is derived by observing the step in pressure that reflects the requested flow step of S50.
At a further step S56, when the pressure $p_1$ reaches a defined threshold, a distinct flow profile is applied to measure at least one corresponding value of the pressure-dependent cuff compliance $C_{cuff}$. This may be achieved by dividing the requested flow $I_{cuff}$ by the detected inflation rate $dp_1/dt$.
At a further step S58, based on the measured values representing compliance $C_{cuff}$, the actual cuff type is identified. Based on the identified type of the cuff, a compliance model describing the cuff compliance $C_{cuff}$ as a function of pressure $p_1$ is obtained. Hence, the pressure $p_1$ may be measured and, consequently, the cuff compliance $C_{cuff}$ can be tracked during the remaining measurement procedure, step S60.
At a further step S62, as a result, defined inflation of the cuff may be started to initiate the blood pressure measurement as such. Inflation of the cuff is performed under control of a feed forward controller. As target inflation rate is requested and processed.
Further, assuming that remaining errors in the system and/or the model based feed forward control approach cause deviations between the target inflation rate and the present inflation rate, at a further step S64, also feedback control is performed which may play a certain part in the control action. Feedback control basically enables an improved target tracking and error compensation. However, in accordance with the present disclosure, feedback control may be primarily regarded as supplemental control in addition to the dominant feed forward control. Preferably, the feedback controller is a relatively slow integral feedback controller as this integral controller exhibits considerably slow responsivity, feedback control does not level off the oscillations in the pressure $p_1$ (and the inflation rate $dp_1/dt$ which equals $dp_2/dt$).

Eventually, at a step S66, the signal of interest may be detected and calculated, particularly a blood pressure indicative signal which is detected at the inflation stage of the cuff based on the detected pressure $p_1$. Hence, the measurement procedure may be accomplished faster than standard NIBP measurements that are based on deflating the cuff.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A blood pressure measurement device (12) comprising:

   - a pressurizing unit (30) arranged to supply an inflation flow, via at least one supply line (32), to a wearable cuff (14) adapted to pressurize a measurement site (16) of a subject of interest, the pressurizing unit (30), the supply line (32) and the wearable cuff (14) forming a pneumatic flow circuit,
   - a pressure detection unit (40) for detecting a cuff pressure ($p_2$) in a direct or mediate fashion, and
   - a control section (50) comprising:

     - a blood pressure determination unit (100) arranged to calculate a blood pressure value based on the cuff pressure ($p_2$) detected by the pressure detection unit (40) during inflation when the cuff pressure ($p_2$) is increased by the pressurizing unit (30) in accordance with a defined pressurizing regime,
     - a feed forward controller (64) arranged to output a target inflation flow ($I_{cuff}$) signal based on a desired value ; of the cuff inflation rate ($dp_2/dt$) or of the cuff pressure ($p_2$) in accordance with the pressurizing regime, and on a model of the pneumatic flow circuit underlying the pressurizing regime, and
     - a feedback controller (62) arranged to minimize the error between the desired value and an actual measured signal for the desired value,

   wherein the output of the feed forward controller (64) and the output of the feedback controller (62) are combined to drive the pressurizing unit (30).

2. The blood pressure measurement device (12) as claimed in claim 1, wherein the feed forward controller (64) is a continuous or quasi-continuous feed forward controller (64) that is arranged to output the target inflation flow ($I_{cuff}$) in a continuous or quasi-continuous fashion, and wherein the feedback controller (62) is arranged to control remaining deviations of the cuff pressure ($p_2$) or inflation rate ($dp_2/dt$) so as to complement the feed forward controller (64), and wherein the combined outputs of the feed forward and feedback controller (62) are converted to an inflation flow set signal that is send to the pressurizing unit (30).

3. The blood pressure measurement device (12) as claimed in claim 2, wherein an update rate of the feed forward and feedback controller (64, 62) is at least 10 Hz, and wherein the feedback controller (62) is a slow feedback controller, wherein the gains of the feedback controller (62) are selected in a range to allow oscillatory pressure signals which are indicative of blood pressure to pass.

4. The blood pressure measurement device as claimed in claim 1, wherein the pneumatic flow circuit is modelled on the basis of a reference pneumatic circuit involving the pressurizing unit (30) and the cuff (14), when attached at the measurement site (16), and a supply line (32) between the pressurizing unit (30) and the cuff (14) which corresponds to a modelled reference line resistance ($R_{tube}$).

5. The blood pressure measurement device (12) as claimed in claim 1, wherein the pressurizing unit (30) is a pump, and wherein the the target inflation flow signal output by the feed forward controller is output in accordance with a flow model with the following equations:

$$(1) \qquad I_{pump}(t) \qquad = f(p_1, V_{pump})$$

$$(2) \qquad p_2(t) \qquad = 1/C_{cuff} \cdot \int_{t0}^{t} Icuff(\tau)d\tau + p2(t_0)$$

$$(3) \qquad \frac{dp_2}{dt} \qquad = 1/ C_{cuff} \cdot I_{cuff}$$

$$(4) \qquad p_1 \qquad = p_2 + I_{pump} \cdot R_{tube}$$

$$(5) \qquad I_{cuff} \qquad \approx I_{tube} \approx I_{pump},$$

wherein $p_1$ represents device pressure, $p_2$ represents cuff pressure, $I_{cuff}$ represents an inflation flow generated by the pressurizing unit (30), $dp_2/dt$ represents the cuff inflation rate, $V_{pump}$ represents an input control signal for the pump, $R_{tube}$ represents line resistance and $C_{cuff}$ represents cuff compliance.

6. The blood pressure measurement device (12) as claimed in claim 5, wherein the control section (50) is arranged to control at least one of the cuff inflation rate ($dp_2/dt$) or the cuff pressure ($p_2$) in a mediate fashion by adapting the inflation flow ($I_{cuff}$) into the cuff (14), wherein the device pressure ($p_1$) is measured during inflation, and wherein cuff compliance ($C_{cuff}$) and line resistance ($R_{tube}$) are determined at least once at the start of the measurement in accordance with the equations as claimed in claim 5.

7. The blood pressure measurement device (12) as claimed in claim 6, wherein the cuff compliance $C_{cuff}$ at the measurement site (16) is pressure dependent, and wherein the cuff compliance ($C_{cuff}$) is modelled as being at least one of linear or non-linear dependent on the cuff pressure ($p_2$).

8. The blood pressure measurement device (12) as claimed in claim 7, wherein an initial cuff compliance ($C_{cuff}$) at a certain initial pressure is determined based on an initial inflation flow value and an initial pressure rate value, and wherein the cuff compliance ($C_{cuff}$) during the remainder of the measurement is determined based on the initial cuff compliance and a continuous or quasi-continuous detection of device pressure ($p_1$) and the compliance model.

9. The blood pressure measurement device (12) as claimed in claim 8, wherein the inflation flow value that is used to measure the cuff compliance is the flow value that is based on the pump flow model.

10. The blood pressure measurement device (12) as claimed in claim 7, wherein line resistance ($R_{tube}$) is determined by applying a defined inflation flow step, and by determining a resulting device pressure step ($p_1$).

11. The blood pressure measurement device (12) as claimed in claim 7, wherein the combined inflation flow set value (84) from the feed forward (64) and feedback controller (62) is converted in a pump voltage, wherein the actual inflation flow into the cuff (14) is measured and wherein an inflation flow feedback controller (90) ensures that the resulting pump voltage ($V_{pump}$) results in the requested inflation flow.

12. The blood pressure measurement device (12) as claimed in claim 7, wherein the combined inflation flow set value (84) from the feed forward controller (64) and the feedback controller (62) is converted in a pump voltage ($V_{pump}$) by a pump flow model that relates the inflation flow ($I_{cuff}$) and cuff pressure ($p_2$) to a pump voltage, such that output of the feed forward controller (64) and the feedback controller (62) have a linear relation with the actual outputted pump flow.

13. A blood pressure measurement method comprising the following steps:

- attaching a wearable cuff (14) which is arranged to pressurize a measurement site (16) of a subject of interest,
- operating a pressurizing unit (30) that is arranged to supply the cuff (14) with an inflation flow of a pressurized fluid via at least one supply line (32), the pressurizing unit (30), the supply line (32) and the wearable cuff (14) forming a pneumatic flow circuit,
- detecting and monitoring a cuff pressure ($p_2$) in a direct or mediate fashion,
- determining a blood pressure value based on the detected cuff pressure ($p_2$) during inflation when the cuff pressure ($p_2$) is increased by the pressurizing unit (30) in accordance with a defined pressurizing regime, and
- controlling inflation of the cuff (14), comprising:

- setting a required inflation flow ($I_{cuff}$) by means of a feed forward controller (64), including outputting a target inflation flow signal based on a desired value of the cuff inflation rate or of the cuff pressure and on a model of the pneumatic flow circuit underlying the pressurizing regime, and
- controlling an actual inflation flow by means of a feedback controller (62), including minimizing the error between the desired value and an actual measured signal for the desired value, wherein the output of the feed forward controller (64) and the output of the feedback controller (62) are combined to drive the pressurizing unit (30).

14. The method as claimed in claim 13, comprising the following steps:

- enabling the pressurizing unit (30) by a defined flow step, including setting a target inflation flow value,
- detecting a pressure step at the pressurizing unit (30) or the cuff (14) in response to the applied flow step,
- calculating line resistance ($R_{tube}$) based on the pressure step and the target inflation flow value,
- determining an initial cuff compliance ($C_{cuff}$) value based on a defined flow value and a pressure rate value,
- identifying an actual cuff type based on the detected cuff compliance ($C_{cuff}$) value,
- tracking the cuff compliance ($C_{cuff}$) based on a compliance estimation model assigned with the actual cuff type and ongoing cuff pressure detection,
- inflating the cuff (14) under control of the feed forward controller (64), wherein a desired inflation rate ($dp_2/dt$) is set by the feed forward controller (64),
- applying feedback control by the feedback controller (62) to adapt the inflation flow ($I_{cuff}$) based on detected deviations between the desired inflation rate ($dp_2/dt$) and an actual inflation rate, and
- calculating blood pressure based on the observed cuff pressure ($p_2$) at the inflation stage.

15. Computer program comprising program code means for causing the control section (50) of the blood pressure measurement device as claimed in any of claims 1-12 to carry out the steps of the method as claimed in claim 13 or 14 when said computer program is carried out on said blood pressure measurement device.

**Patentansprüche**

1. Ein Blutdruckmessgerät (12), das Folgendes umfasst:

eine Druckbeaufschlagungseinheit (30), die einer tragbaren Manschette (14) über mindestens eine Versorgungsleitung (32) einen Aufblasstrom zuführt, mit der die Messstelle (16) eines zu untersuchenden Objekts mit Druck beaufschlagt, wobei die Druckbeaufschlagungseinheit (30), die Versorgungsleitung (32) und die tragbare Manschette (14) einen pneumatischen Strömungskreislauf bilden,
eine Druckerkennungseinheit (40) zum Erkennen des Manschettendrucks ($p_2$) auf direkte oder mittelbare Weise, und
eine Steuerungsgruppe (50), die Folgendes umfasst:

eine Blutdruckbestimmungseinheit (100), die den Blutdruckwert anhand des Manschettendrucks ($p_2$) berechnet, der von der Druckerkennungseinheit (40) beim Aufblasen ermittelt wird, wenn der Manschettendruck ($p_2$) von der Druckbeaufschlagungseinheit (30) anhand des definierten Druckbeaufschlagungsprogramms erhöht wird,

einen Vorschubregler (64), der ein Soll-Aufblasstromsignal ($I_{cuff}$) ausgibt, das auf dem gewünschten Wert der Manschettenaufblasrate ($dp_2/dt$) oder des Manschettendrucks ($p_2$) in Übereinstimmung mit dem Druckbeaufschlagungsprogramm sowie einem Modell des dem Druckbeaufschlagungsprogramm zugrunde liegenden pneumatischen Strömungskreislaufs beruht, und

einen Rückkopplungsregler (62), der die Abweichung zwischen dem Sollwert und dem tatsächlich für den Sollwert gemessenen Signal minimiert,

wobei die Ausgaben des Vorschubreglers (64) und des Rückkopplungsreglers (62) kombiniert werden, um die Druckbeaufschlagungseinheit (30) anzutreiben.

2. Das Blutdruckmessgerät (12) gemäß Anspruch 1, wobei es sich beim Vorschubregler (64) um einen kontinuierlichen oder quasi-kontinuierlichen Vorschubregler (64) handelt, der den Ziel-Aufblasstrom ($I_{cuff}$) in einer kontinuierlichen oder quasi-kontinuierlichen Weise ausgibt, und wobei der Rückkopplungsregler (62) verbleibende Abweichungen des Manschettendrucks ($p_2$) oder der Aufblasrate ($dp_2/dt$) so regelt, dass er den Vorschubregler (64) ergänzt, und wobei die kombinierten Ausgaben des Vorschub- und des Rückkopplungsreglers (62) in ein Aufblasstrom-Sollsignal umgewandelt werden, das an die Druckbeaufschlagungseinheit (30) gesendet wird.

3. Das Blutdruckmessgerät (12) gemäß Anspruch 2, wobei die Aktualisierungsrate des Vorschub- und des Rückkopplungsreglers (64, 62) mindestens 10 Hz beträgt, und wobei es sich beim Rückkopplungsregler (62) um einen langsamen Rückkopplungsregler handelt, und wobei die Verstärkung des Rückkopplungsreglers (62) in einem Bereich liegt, den die oszillatorischen Drucksignale, die den Blutdruck angeben, durchlaufen können.

4. Das Blutdruckmessgerät gemäß Anspruch 1, wobei der pneumatische Strömungskreislauf beruhend auf einem pneumatischen Referenzkreislauf modelliert wird, der die Druckbeaufschlagungseinheit (30), die an der Messstelle (16) angebrachte Manschette (14) und eine Versorgungsleitung (32) zwischen der Druckbeaufschlagungseinheit (30) und der Manschette (14) umfasst, und der einem modellierten Referenzleitungswiderstand ($R_{tube}$) entspricht.

5. Das Blutdruckmessgerät (12) gemäß Anspruch 1, wobei es sich bei der Druckbeaufschlagungseinheit (30) um eine Pumpe handelt, und wobei das vom Vorschubregler ausgegebene Soll-Aufblasstromsignal anhand eines Strömungsmodells mit den folgenden Gleichungen ausgegeben wird:

$$(1) \quad I_{pump}(t) = f(p_1, V_{pump})$$

$$(2) \quad p_2(t) = 1/C_{cuff} \cdot \int_{t0}^{t} Icuff(\tau)d\tau + p2(t_0)$$

$$(3) \quad \frac{dp_2}{dt} = 1/C_{cuff} \cdot I_{cuff}$$

$$(4) \quad p_1 = p_2 + I_{pump} \cdot R_{tube}$$

$$(5) \quad I_{cuff} \approx I_{tube} \approx I_{pump},$$

wobei $p_1$ dem Gerätedruck, $p_2$ dem Manschettendruck, $I_{cuff}$ dem von der Druckeinheit (30) erzeugten Aufblasstrom, $dp_2/dt$ der Manschettenaufblasrate, $V_{pump}$ dem Eingangssteuerungssignal für die Pumpe, $R_{tube}$ dem Leitungswiderstand und $C_{cuff}$ der Manschettenkonformität entspricht.

6. Das Blutdruckmessgerät (12) gemäß Anspruch 5, wobei die Steuerungsgruppe (50) mindestens entweder die Manschettenaufblasrate ($dp_2/dt$) oder den Manschettendruck ($p_2$) auf mittelbare Weise steuert, indem der Aufblasstrom ($I_{cuff}$) für die Manschette (14) anpasst, und wobei der Gerätedruck ($p_1$) beim Aufblasen gemessen wird, und wobei die Manschettenkonformität ($C_{cuff}$) und der Leitungswiderstand ($R_{tube}$) zumindest einmal zu Beginn der Messung anhand der Gleichungen in Anspruch 5 bestimmt werden.

7. Das Blutdruckmessgerät (12) gemäß Anspruch 6, wobei die Manschettenkonformität $C_{cuff}$ an der Messstelle (16) druckabhängig ist, und wobei die Manschettenkonformität ($C_{cuff}$) so modelliert ist, dass sie mindestens entweder linear oder nicht-linear vom Manschettendruck ($p_2$) abhängt.

8. Das Blutdruckmessgerät (12) gemäß Anspruch 7, wobei die anfängliche Manschettenkonformität ($C_{cuff}$) bei einem bestimmten Anfangsdruck anhand des anfänglichen Aufblasstromwerts und des anfänglichen Druckratenwerts bestimmt wird, und wobei die Manschettenkonformität ($C_{cuff}$) im Verlauf der übrigen Messung anhand der anfänglichen Manschettenkonformität und des kontinuierlichen oder quasi-kontinuierlichen Erfassens des Gerätedrucks ($p_1$) und des Konformitätsmodells bestimmt wird.

9. Das Blutdruckmessgerät (12) gemäß Anspruch 8, wobei es sich beim Aufblasstromwert für das Messen der Manschettenkonformität um den Stromwert handelt, der auf dem Pumpenstrommodell beruht.

10. Das Blutdruckmessgerät (12) gemäß Anspruch 7, wobei der Leitungswiderstand ($R_{tube}$) durch das Übernehmen eines definierten Aufblasstromschritts sowie durch Ermitteln des resultierenden Gerätedruckschritts ($p_1$) bestimmt wird.

11. Das Blutdruckmessgerät (12) gemäß Anspruch 7, wobei der kombinierte Aufblasstrom-Sollwert (84) vom Vorschubregler (64) und Rückkopplungsregler (62) in eine Pumpenspannung umgewandelt wird, und wobei der tatsächliche Aufblasstrom für die Manschette (14) gemessen wird, und wobei ein Aufblasstrom-Rückkopplungsregler (90) sicherstellt, dass die resultierende Pumpenspannung ($V_{pump}$) den gewünschten Aufblasstrom ergibt.

12. Das Blutdruckmessgerät (12) gemäß Anspruch 7, wobei der kombinierte Aufblasstrom-Sollwert (84) vom Vorschubregler (64) und Rückkopplungsregler (62) durch ein Pumpenstrommodell, das den Aufblasstrom ($I_{cuff}$) und den Manschettendruck ($p_2$) zu einer Pumpenspannung in Beziehung setzt, in eine Pumpenspannung ($V_{pump}$) umgewandelt wird, sodass die Ausgabe des Vorschubreglers (64) und des Rückkopplungsreglers (62) in einer linearen Beziehung zum tatsächlich ausgegebenen Pumpenstrom steht.

13. Eine Blutdruckmessmethode die folgende Schritte umfasst:

Anbringen einer tragbaren Manschette (14), die die Messstelle (16) am zu untersuchenden Objekt mit Druck beaufschlagt,
Betreiben einer Druckbeaufschlagungseinheit (30), die die Manschette (14) über mindestens eine Versorgungsleitung (32) mit einem Aufblasstrom einer unter Druck stehenden Flüssigkeit versorgt, wobei die Druckbeaufschlagungseinheit (30), die Versorgungsleitung (32) und die tragbare Manschette (14) einen pneumatischen Strömungskreislauf bilden,
Erkennen und Überwachen des Manschettendrucks ($p_2$) auf direkte oder mittelbare Weise,
Ermitteln des Blutdruckwerts anhand des beim Aufblasen ermittelten Manschettendrucks ($p_2$), wenn der Manschettendruck ($p_2$) von der Druckbeaufschlagungseinheit (30) anhand des definierten Druckbeaufschlagungsprogramms erhöht wird, und
Regeln des Aufblasens der Manschette (14) mithilfe der folgenden Schritte:

Einstellen des erforderlichen Aufblasstroms ($I_{cuff}$) mit einem Vorschubregler (64) sowie Ausgeben eines Soll-Aufblasstromsignals anhand des Sollwerts der Manschettenaufblasrate oder des Manschettendrucks und eines Modells des pneumatischen Strömungskreislaufs, das dem Druckbeaufschlagungsprogramm zugrunde liegt, und
Regeln des tatsächlichen Aufblasstroms mit einem Rücckopplungsregler (62) sowie Minimieren der Abweichung zwischen dem Sollwert und dem tatsächlich für den Sollwert gemessenen Signal, wobei die Ausgaben des Vorschubreglers (64) und des Rückkopplungsreglers (62) kombiniert werden, um die Druckbeaufschlagungseinheit (30) anzutreiben.

14. Die Methode gemäß Anspruch 13, die folgende Schritte umfasst:

Aktivieren der Druckbeaufschlagungseinheit (30) durch einen definierten Stromschritt sowie Festlegen eines Ziel-Aufblasstromwerts,
Erkennen eines Druckschritts an der Druckbeaufschlagungseinheit (30) oder der Manschette (14) als Reaktion auf den umgesetzten Stromschritt,
Berechnen des Leitungswiderstands ($R_{tube}$) anhand des Druckschritts und des Zielwerts für den Aufblasstrom, Ermitteln des anfänglichen Manschettenkonformitätswerts ($C_{cuff}$) anhand eines definierten Stromwerts und eines Druckratenwerts,

Ermitteln des tatsächlichen Manschettentyps anhand des ermittelten Manschettenkonformitätswerts ($C_{cuff}$),

Nachverfolgen der Manschettenkonformität ($C_{cuff}$) anhand eines Konformitätsschätzmodells, das dem tatsächlichen Manschettentyp und der laufenden Manschettendruckerkennung zugeordnet ist,

Aufblasen der vom Vorschubregler (64) geregelten Manschette (14), wobei die gewünschte Aufblasrate ($dp_2/dt$) vom Vorschubregler (64) eingestellt wird, Übernehmen der Rückkopplungsregelung durch den Rücckopplungsregler (62), um den Aufblasstrom ($I_{cuff}$) anhand der ermittelten Abweichungen zwischen der gewünschten Aufblasrate ($dp_2/dt$) und der tatsächlichen Aufblasrate anzupassen, und

Berechnen des Blutdrucks anhand des ermittelten Manschettendrucks ($p_2$) in der Aufblasphase.

15. Ein Computerprogramm mit Programmcode, das die Steuerungsgruppe (50) des Blutdruckmessgeräts gemäß einer der Ansprüche 1 bis 12 dazu veranlasst, die Schritte der Methode gemäß der Ansprüche 13 oder 14 auszuführen, wenn das Computerprogramm auf dem Blutdruckmessgerät ausgeführt wird.

## Revendications

1. Dispositif de mesure de la pression artérielle (12) comprenant :

   une unité de pressurisation (30) conçue pour fournir un écoulement de gonflage, par l'intermédiaire d'au moins une conduite d'alimentation (32), à un brassard (14) portable conçu pour pressuriser un site de mesure (16) d'un sujet d'intérêt, l'unité de pressurisation (30), la conduite d'alimentation (32) et le brassard (14) portable formant un circuit d'écoulement pneumatique,
   une unité de détection de pression (40) pour détecter la pression du brassard ($p_2$) de manière directe ou intermédiaire, et
   une section de commande (50) comprenant :

   une unité de détermination de la pression artérielle (100) conçue pour calculer une valeur de pression artérielle en fonction de la pression du brassard ($p_2$) détectée par l'unité de détection de pression (40) lors du gonflage lorsque la pression du brassard ($p_2$) est augmentée par l'unité de pressurisation (30) selon un régime de pressurisation défini,
   un dispositif de commande par anticipation (64) conçu pour sortir un signal de gonflage ($I_{cuff}$) cible en fonction d'une valeur souhaitée du taux de gonflage du brassard ($dp_2/dt$) ou de la pression du brassard ($p_2$) selon le régime de pressurisation, et d'un modèle du circuit d'écoulement pneumatique sous-jacent au régime de pressurisation, et
   un dispositif de commande par rétroaction (62) conçu pour minimiser l'erreur entre la valeur souhaitée et un signal mesuré réel pour la valeur souhaitée, dans lequel la sortie du dispositif de commande par anticipation (64) et la sortie du dispositif de commande par rétroaction (62) sont combinées pour entraîner l'unité de pressurisation (30).

2. Dispositif de mesure de la pression artérielle (12) selon la revendication 1, dans lequel le dispositif de commande par anticipation (64) est un dispositif de commande par anticipation continu ou quasi-continu (64), lequel est conçu pour sortir l'écoulement de gonflage ($I_{cuff}$) cible d'une manière continue ou quasi continue, et dans lequel le dispositif de commande par rétroaction (62) est conçu pour commander les écarts restants de la pression du brassard ($p_2$) ou du taux de gonflage ($dp_2/dt$) de manière à compléter le dispositif de commande par anticipation (64), et dans lequel les sorties combinées du dispositif de commande par anticipation et par rétroaction (62) sont converties en un signal de réglage de l'écoulement de gonflage, lequel est envoyé à l'unité de pressurisation (30).

3. Dispositif de mesure de la pression artérielle (12) selon la revendication 2, dans lequel une fréquence de mise à jour du dispositif de commande par anticipation et par rétroaction (64, 62) est d'au moins 10 Hz, et dans lequel le dispositif de commande par rétroaction (62) est un dispositif de commande par rétroaction lente, dans lequel les gains du dispositif de commande par rétroaction (62) sont sélectionnés dans une plage pour permettre aux signaux de pression oscillatoires, lesquels sont indicatifs de la pression sanguine, d'être supérieurs.

4. Dispositif de mesure de la pression artérielle selon la revendication 1, dans lequel le circuit pneumatique d'écoulement est modélisé en fonction d'un circuit pneumatique de référence impliquant l'unité de pressurisation (30) et le brassard (14), lorsqu'il est fixé au site de mesure (16), et d'une conduite d'alimentation (32) entre l'unité de pressurisation (30) et le brassard (14), laquelle correspond à une résistance de conduite de référence modélisée ($R_{tube}$) .

**5.** Dispositif de mesure de la pression artérielle (12) selon la revendication 1, dans lequel l'unité de pressurisation (30) est une pompe, et dans lequel le signal d'écoulement de gonflage cible sorti par le dispositif de commande par anticipation est sorti selon un modèle d'écoulement présentant les équations suivantes :

$$(1) \quad I_{pump}(t) = f(p_1, V_{pump})$$

$$(2) \quad p_2(t) = 1/C_{cuff} \cdot f^t_{t0} Icuff(\tau) d\tau + p2(t_0)$$

$$(3) \quad \frac{dp_2}{dt} = 1/C_{cuff} \cdot I_{cuff}$$

$$(4) \quad p_1 = p_2 + I_{pump} \cdot R_{tube}$$

$$(5) \quad I_{cuff} \approx I_{tube} \approx I_{pump},$$

$p_1$ représentant la pression du dispositif, $p_2$ représentant la pression du brassard, $I_{cuff}$ représentant un écoulement de gonflage généré par l'unité de pressurisation (30), $dp_2/dt$ représentant le taux de gonflage du brassard, $V_{pump}$ représentant un signal de commande d'entrée pour la pompe, $R_{tube}$ représentant la résistance de conduite et $C_{cuff}$ représentant la conformité du brassard.

**6.** Dispositif de mesure de la pression artérielle (12) selon la revendication 5, dans lequel une section de commande (50) est conçue pour commander le taux de gonflage du brassard ($dp_2/dt$) et/ou la pression du brassard ($p_2$) de manière intermédiaire par adaptation de l'écoulement de gonflage ($I_{cuff}$) dans le brassard (14), dans lequel la pression du dispositif ($p_1$) est mesurée lors du gonflage, et dans lequel la conformité du brassard ($C_{cuff}$) et la résistance de conduite ($R_{tube}$) sont déterminées au moins une fois au début de la mesure selon les équations selon la revendication 5.

**7.** Dispositif de mesure de la pression artérielle (12) selon la revendication 6, dans lequel la conformité du brassard ($C_{cuff}$) au site de mesure (16) dépend de la pression, et dans lequel la conformité du brassard ($C_{cuff}$) est modélisée comme étant dépendante de manière linéaire et/ou non linéaire de la pression de brassard ($p_2$).

**8.** Dispositif de mesure de la pression artérielle (12) selon la revendication 7, dans lequel une conformité initiale du brassard ($C_{cuff}$) à une certaine pression initiale est déterminée en fonction d'une valeur d'écoulement de gonflage initiale et d'une valeur de taux de pression initiale, et dans lequel la conformité du brassard ($C_{cuff}$) lors du reste de la mesure est déterminée en fonction de la conformité initiale du brassard et d'une détection continue ou quasi continue de la pression ($p_1$) du dispositif et du modèle de conformité.

**9.** Dispositif de mesure de la pression artérielle (12) selon la revendication 8, dans lequel la valeur d'écoulement de gonflage, laquelle est utilisée pour mesurer la conformité du brassard est la valeur d'écoulement, laquelle est basée sur le modèle d'écoulement de pompe.

**10.** Dispositif de mesure de la pression artérielle (12) selon la revendication 7, dans lequel la résistance de conduite ($R_{tube}$) est déterminée par application d'une étape d'écoulement de gonflage définie, et par détermination d'une étape de pression du dispositif résultante ($p_1$).

**11.** Dispositif de mesure de la pression artérielle (12) selon la revendication 7, dans lequel la valeur de réglage du gonflage combinée (84) provenant du dispositif de commande par anticipation (64) et du dispositif de commande par rétroaction (62) est convertie en une tension de pompe, dans lequel l'écoulement de gonflage réel dans le brassard (14) est mesuré et dans lequel un dispositif de commande par rétroaction de l'écoulement de gonflage (90) garantit que la tension de pompe (V$_{pump}$) résultante entraîne le gonflage demandé.

**12.** Dispositif de mesure de la pression artérielle (12) selon la revendication 7, dans lequel la valeur de réglage de l'écoulement de gonflage combiné (84) provenant du dispositif de commande par anticipation (64) et du dispositif de commande par rétroaction (62) est convertie en une tension de pompe (V$_{pump}$) selon un modèle d'écoulement de pompe, lequel relie l'écoulement de gonflage (I$_{cuff}$) et la pression du brassard (p$_2$) à une tension de pompe de telle sorte que la sortie du dispositif de commande par anticipation (64) et du dispositif de commande par rétroaction (62) présente une relation linéaire avec l'écoulement de pompe réellement sorti.

**13.** Procédé de mesure de la pression artérielle comprenant les étapes suivantes :

la fixation d'un brassard (14) portable, lequel est conçu pour pressuriser un site de mesure (16) d'un sujet d'intérêt, l'actionnement d'une unité de pressurisation (30), laquelle est conçue pour fournir au brassard (14) un écoulement de gonflage d'un fluide pressurisé par l'intermédiaire d'au moins une conduite d'alimentation (32), l'unité de pressurisation (30), la conduite d'alimentation (32) et le brassard (14) portable formant un circuit d'écoulement pneumatique, la détection et la surveillance d'une pression du brassard (p$_2$) de manière directe ou intermédiaire, la détermination d'une valeur de pression artérielle en fonction de la pression de brassard (p$_2$) détectée lors du gonflage lorsque la pression de brassard (p$_2$) est augmentée par l'unité de pressurisation (30) selon un régime de pressurisation défini, et la commande du gonflage du brassard (14), comprenant :

le réglage d'un écoulement de gonflage (I$_{cuff}$) requis au moyen d'un dispositif de commande par anticipation (64), comprenant la sortie d'un signal d'écoulement de gonflage cible en fonction d'une valeur souhaitée du taux de gonflage du brassard ou de la pression du brassard et d'un modèle du circuit d'écoulement pneumatique sous-jacent au régime de pressurisation, et la commande d'un écoulement de gonflage réel au moyen d'un dispositif de commande par rétroaction (62), comprenant la minimisation de l'erreur entre la valeur souhaitée et un signal mesuré réel pour la valeur souhaitée, dans lequel la sortie du dispositif de commande par anticipation (64) et la sortie du dispositif de commande par rétroaction (62) sont combinées pour entraîner l'unité de pressurisation (30).

**14.** Procédé selon la revendication 13, comprenant les étapes suivantes :

l'activation de l'unité de pressurisation (30) au moyen d'une étape d'écoulement définie, comprenant le réglage d'une valeur d'écoulement de gonflage cible, la détection d'une étape de pression au niveau de l'unité de pressurisation (30) ou du brassard (14) en réponse à l'étape d'écoulement appliquée, le calcul de la résistance de conduite (R$_{tube}$) en fonction de l'étape de pression et de la valeur cible de l'écoulement de gonflage, la détermination d'une valeur initiale de conformité du brassard (C$_{cuff}$) en fonction d'une valeur d'écoulement définie et d'une valeur d'écoulement de pression, l'identification d'un type de brassard réel en fonction de la valeur de conformité du brassard (C$_{cuff}$) détectée, le suivi de la conformité du brassard (C$_{cuff}$) en fonction d'un modèle d'estimation de la conformité attribué au type réel de brassard et de la détection de la pression du brassard en cours, le gonflage du brassard (14) sous la commande du dispositif de commande par anticipation (64), dans lequel un taux de gonflage (dp$_2$/dt) souhaité est réglé par le dispositif de commande par anticipation (64), l'application d'une commande par rétroaction par le dispositif de commande par rétroaction (62) pour adapter l'écoulement de gonflage (I$_{cuff}$) en fonction des écarts détectés entre le taux de gonflage (dp$_2$/dt) souhaité et un taux de gonflage réel, et le calcul de la pression artérielle en fonction de la pression du brassard (p$_2$) observée à l'étape de gonflage.

**15.** Programme informatique comprenant un moyen de code de programme pour amener la section de commande (50) du dispositif de mesure de la pression artérielle selon l'une quelconque des revendications 1 à 12 à mettre en œuvre les étapes du procédé selon la revendication 13 ou 14 lorsque ledit programme informatique est exécuté sur ledit dispositif de mesure de la pression artérielle.

FIG.1

FIG.2

FIG.3

$$p_1 = p_2 + I_c * R_t$$

$$\Delta p_1 = I_c * R_t$$

$$p_2 = 1/C * \int I_c \, dt$$

FIG.4

$C_A$

$C_B$

$C_1$

$p_1$

FIG.5

FIG.6

FIG.7

FIG.8

FIG.9

FIG.10

**REFERENCES CITED IN THE DESCRIPTION**

**Patent documents cited in the description**

- US 20140309541 A1
- US 2014257116 A1
- US 2014316290 A1
- US 2012323128 A1
- JP 2006129920 A
- US 2015230718 A1